# EUROPEAN PATENT SPECIFICATION

(11) **EP 3 478 282 B1**
(45) Date of publication and mention of the grant of the patent: **29.07.2020**
(21) Application number: 17755233.8
(22) Date of filing: 30.06.2017
(51) Int. Cl.: A61K 31/353, A61K 31/395, A61K 47/54, A61P 35/00, A61P 35/02, A61K 45/06, A61K 31/706

(54) **PHARMACEUTICAL COMPOSITION OF [ (3-HYDROXY-4 -PYRON-2-YL) METHYL]-AMINE DERIVATIVES AND DNA DEMETHYLATING AGENTS AND THEIR USE AS ANTI-NEOPLASTIC DRUGS**
PHARMAZEUTISCHE ZUSAMMENSETZUNG AUS [(3-HYDROXY-4-PYRON-2-YL)METHYL]-AMIN-DERIVATEN UND DNA-DEMETHYLIERUNGSMITTEL SOWIE DEREN VERWENDUNG ALS ANTINEOPLASTISCHE WIRKSTOFFE
COMPOSITION PHARMACEUTIQUE À BASE DE DÉRIVÉS DE [(3-HYDROXY-4-PYRON-2-YL)MÉTHYL]AMINE ET D'AGENTS DE DÉMÉTHYLATION DE L'ADN ET SON UTILISATION À TITRE D'ANTI-NÉOPLASIQUE

(30) Priority: 30.06.2016 IT UA20164811
(43) Date of publication of application: 08.05.2019
(73) Proprietor: Università Degli Studi Di Urbino "Carlo Bo", 61029 Urbino (IT)
(72) Inventor: FANELLI, Mirco, 61029 URBINO (IT); FUSI, Vieri, 61029 URBINO (IT)
(74) Representative: Fiussello, Francesco
(86) International application number: PCT/IB2017/053958
(87) International publication number: WO 2018/002896

(56) References cited:
- WO-A1-2010/061282
- S AMATORI ET AL: "Malten, a new synthetic molecule showing in vitro antiproliferative activity against tumour cells and induction of complex DNA structural alterations", BRITISH JOURNAL OF CANCER, vol. 103, no. 2, 22 June 2010 (2010-06-22) , pages 239-248, XP055361658, GB ISSN: 0007-0920, DOI: 10.1038/sj.bjc.6605745
- CLARA GUERZONI ET AL: "An aza-macrocycle containing maltolic side-arms (maltonis) as potential drug against human pediatric sarcomas", BMC CANCER, BIOMED CENTRAL, LONDON, GB, vol. 14, no. 1, 27 February 2014 (2014-02-27), page 137, XP021178149, ISSN: 1471-2407, DOI: 10.1186/1471-2407-14-137
- SANTINI V ET AL: "Changes in DNA methylation in neoplasia: Pathophysiology and therapeutic implications", ANNALS OF INTERNAL MEDICINE 20010403 US, vol. 134, no. 7, 3 April 2001 (2001-04-03) , pages 573-586, XP002280241, ISSN: 0003-4819

## Description

### TECHNICAL FIELD

The present invention concerns poly-alkyl-bis-maltolic molecules and in particular [(3-hydroxy-4-pyron-2-yl)methyl]-amine derivatives and their use as anti-neoplastic drugs in combination with DNA demethylating agents. In particular, for the preparation of a medicament for the treatment of neoplastic pathologies.

### PRIOR ART

Despite the important progress made in the treatment of neoplastic pathologies, the survival of patients affected by tumour remains, in many cases, extremely limited (e.g. Glioblastoma Multiforme - GBM, Malignant Pleural Mesothelioma - MPM). The absence of specific therapies, resistance to the chemotherapeutic drugs currently used and the high possibility of recurrence represent the main causes of failure in the treatment of many neoplasias. In addition, the majority of the chemotherapeutic drugs currently used are cytotoxic drugs, which are known to cause significant side effects.

Studies on two maltol-derived molecules named Malten, i.e. [N, N'-bis[(3-hydroxy-4-Pyron-2-yl)methyl]-N,N'-dimethylethylenediamine] and Maltonis [4(N), 10(N) -bis [(3-hydroxy-4-Pyron-2-yl) methyl]-1,7-dimethyl-1, 4, 7, 10 tetraazacyclododecane] are already known, in particular Amatori S, Bagaloni I, Macedi E, Formica M, Giorgi L, Fusi V, Fanelli M. Malten, "a new synthetic molecule showing in vitro antiproliferative activity against tumour cells and induction of complex DNA structural alterations" Br J Cancer. 2010; 103(2) :239-248. doi: 10.1038/sj.bjc.6605745; Amatori S, Ambrosi G, Fanelli M, Formica M, Fusi V, Giorgi L, Macedi E, Micheloni M, Paoli P, Pontellini R, Rossi P. Synthesis, basicity, "structural characterization, and biochemical properties of two [(3-hydroxy-4-pyron-2-yl)methyl]amine derivatives showing antineoplastic features" J Org Chem. 2012;77(5):2207-2218. doi: 10.1021/jo202270j e Guerzoni C, Amatori S, Giorgi L, Manara MC, Landuzzi L, Lollini P-L, Tassoni A, Balducci M, Manfrini M, Pratelli L, Serra M, Picci P, Magnani M, Fusi V, Fanelli M, Scotlandi K (2014). An aza-macrocycle containing maltolic side-arms (maltonis) as potential drug against human pediatric sarcomas BMC Cancer 14:137. doi:10.1186/1471-2407-14-137.

Both the chemical agents belong to the class of highly versatile molecules of the hydroxypyrons, which include compounds having antiproliferative activity against a wide range of tumour cells, alone or preferably in combination with metals.

In particular the compound 3-hydroxy-2-methyl-4-pyron (maltol) is a natural compound used in food products, beverages, tobacco, beer and cosmetics due to its flavour and antioxidant properties.

The maltol and its derivatives reported above show anti-neoplastic activity attributed to the formation of reactive oxygen species (ROS) and properties of coordination towards metallic ions.

For this reason, ligands containing maltol have been developed and exploited as potential new metal-based antitumour drugs. The anti-neoplastic potential was originally verified for Malten, a molecule belonging to this class of poly-alkylamino-bis-maltolic compounds, in various tumour histotypes.

In particular the effect of Malten was studied in eight different neoplastic cell models derived from tumours of both hematopoietic origin and solid tissues such as cancer of the cervix, glioblastoma, pleural mesothelioma and alveolar rhabdomyosarcoma, the latter being the most sensitive histotype with an IC50 two or three times lower than the other models.

On the basis of the above, therefore, research is currently directed at the identification of drugs that are more efficient and/or selective vis-à-vis the tumour cells.

More recently a molecule named Maltonis has been selected and characterized, belonging to the class of poly-alkylamino-bis-maltolic compounds, which has proved effective both in vitro and in vivo vis-à-vis Ewing's sarcoma.

### SUMMARY OF THE INVENTION

The object of the present invention is therefore to obtain more efficient and/or selective compounds vis-à-vis the tumour cells.

According to the present invention said object is achieved by a compound according to claim 1.

### BRIEF DESCRIPTION OF THE FIGURES

For a better understanding of the present invention, it is now described also with reference to the attached figures which illustrate:
- figure 1 a series of graphs that illustrate the combination of some examples of molecules according to the present invention; and
- figure 2 a further series of graphs which illustrate the combination of some examples of molecules according to the present invention,
- figure 3 a series of graphs which illustrate the combination of some examples of molecules according to the present invention; and
- figure 4 a further series of graphs which illustrate the combination of some examples of molecules according to the present invention.

### DETAILED DESCRIPTION OF THE INVENTION

By combined preparation we mean that the composites can be used in combination simultaneously or sequentially both in the same pharmaceutical formulation or in a different pharmaceutical formulation, for treatment of the same pathology.

By pharmaceutical composition we mean not only one single pharmaceutical formulation comprising two different compounds, but also that the two compounds can be present in different pharmaceutical formulations and can be used for combined preparation as defined above and therefore for simultaneous or sequential administration. Merely by way of example, a first pharmaceutical formulation may therefore contain a first compound, for example comprising a compound according to formula I below, and in a second pharmaceutical formulation a second compound. The second pharmaceutical formulation is administered sequentially and after a certain time from the first pharmaceutical formulation. Alternatively, the two compounds can be present in the same pharmaceutical formulation and be administered simultaneously.

Compounds comprising compounds with formula I are the subject of the present invention: 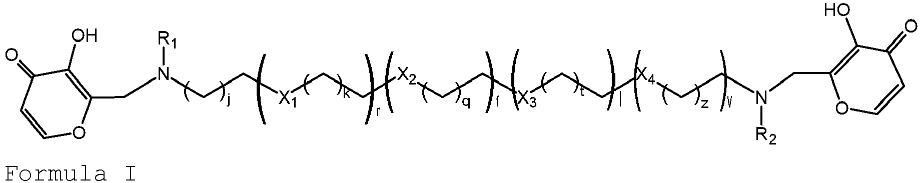 Where:
X₁, X₂, X₃, X₄ = NR₃, O
R₃ = H, CₘH₂ₘ₊₁, m = 1, 2, 3, 4, 5, 6
n, f, 1, v = 0,1
j, k, q, t, z = 1, 2
R₁, R₂ = CₘH₂ₘ₊₁, m = 1, 2, 3, 4, 5, 6 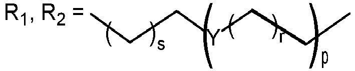 or alternatively form a cycle and have the following meaning:
   where:
   Y = NR₃, O
   R₃ = H, CₘH₂ₘ₊₁, m = 1, 2, 3, 4, 5, 6
   p= 0, 1, 2, 3, 4;
   s= 0, 1, 2
   r= 1, 2
   and their pharmaceutically acceptable salts in combination with DNA demethylating agents and their pharmaceutically acceptable salts in particular for the preparation of a medicament and more preferably for combined preparation for the treatment of a pathology.

By DNA demethylating agents, in the context of the present invention we mean compounds able to inhibit methylation, causing expression of the silenced genes.

Preferably the demethylating agents are analogues of cytidine, 5-aza-cytidine (AZA) and 5-aza-2'-deoxycytidine (DAC). Generally the DNA demethylating agents act by binding with the enzymes that catalyze the methylation reaction.

In combination with the DNA demethylating agents, the compounds with formula II are particularly preferred: 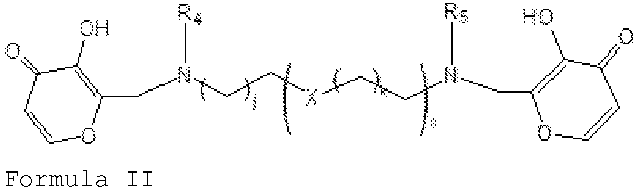 where
X = NR₃, O
R₃ = H, CₘH₂ₘ₊₁, m = 1, 2, 3, 4, 5, 6
n = 0, 1, 2, 3, 4
j, k = 1, 2
R₄, R₅ = CₘH₂ₘ₊₁, m = 1, 2, 3, 4, 5, 6.

The compounds with formula III are also preferred: 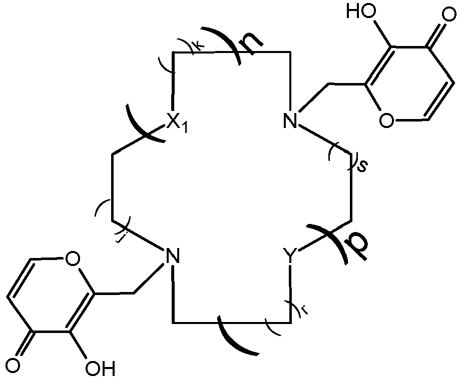 where:
X₁, Y = NR₃, O
R₃ = H, CₘH₂ₘ₊₁, m = 1, 2, 3, 4, 5, 6
n = 0, 1, 2, 3
j, s = 0, 1, 2
p= 0, 1, 2, 3
r, k= 1, 2
and their pharmaceutically acceptable salts.

Among the compounds with formula I, examples of R₃ C1-C6 aliphatic substituents are methyl, ethyl, propyl, isopropyl, butyl, sec-butyl, ter-butyl, pentyl and hexyl.

Among the compounds with formula II, those in which X is N-CH3, and n is 0 or 1 are preferred.

Among the compounds with formula III, the preferred ones are those in which X₁ and Y are NH, N-methyl and N-ethyl or alternatively in which X or Y are equal to O so as to obtain poly-oxa-aza crown ether compounds. The parameters n, p can be modulated by inserting multiple ethers in amine groups obtaining compounds also with 8 functional groups. The parameters j, k, can be varied in order to separate these functions with ethyl or propyl aliphatic chains. By varying several parameters, it is possible to modulate the dimension of the ring starting from the smallest 1,4,7-cyclononanes substituted.

Among the compounds of formula III, a first group of preferred compounds comprises those based on macrocycles of cyclododecanes in which X and Y are N-CH3 or O, and n = k = j = p = s = r = 1 (all ethyl chains).

Persons skilled in the art are able to synthesize and characterize all the compounds with formula I, II, III, carrying out appropriate substitutions in the various groups. The pharmaceutically acceptable salts are all those organic and inorganic salts capable of salifying the base centres present and which do not have a toxic effect or other undesired effects.

The following compounds are particularly preferred:

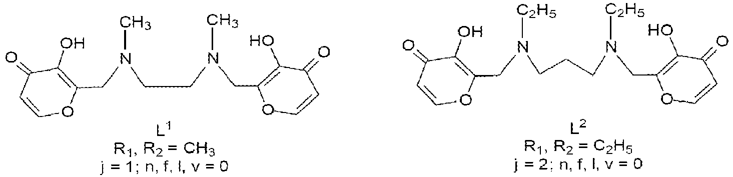

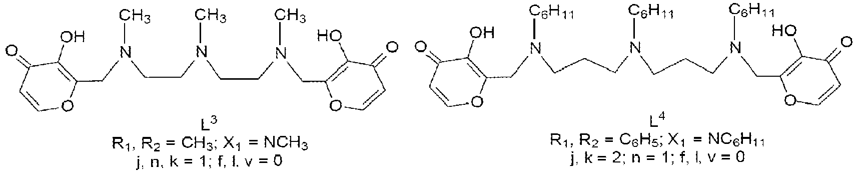

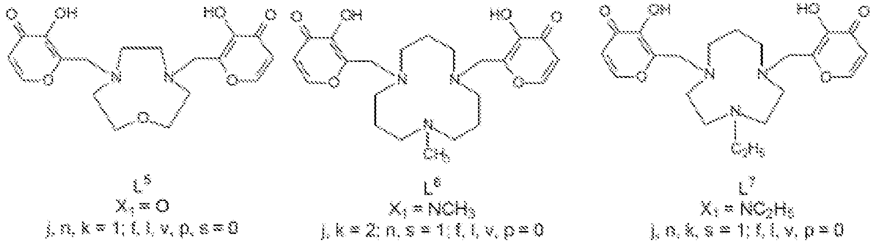

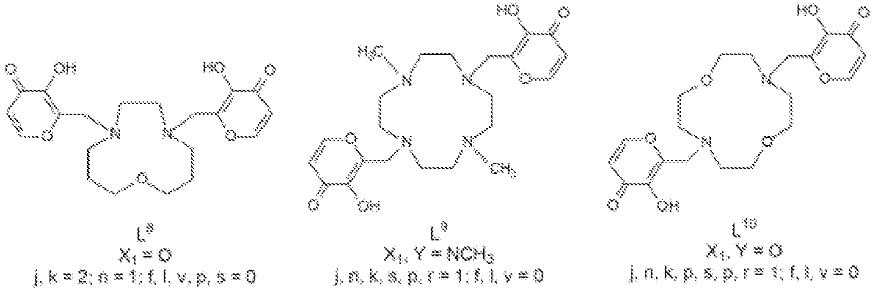

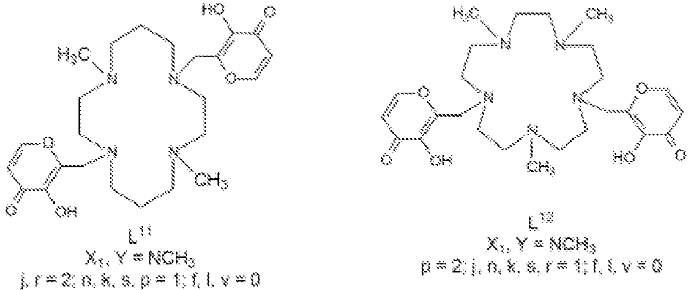

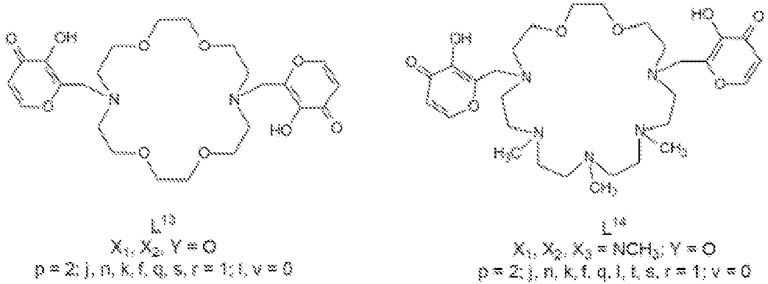

Furthermore, the N,N'-bis[(3-hydroxy-4-pyron-2-yl)methyl]-N,N'-dimethylethylenediamine (L¹) and 4(N),10(N)- bis[(3-hydroxy-4-pyron-2-il)methyl]-1,7-dimethyl-1,4,7,10-tetraazacyclododecane (L⁹) compounds are particularly preferred.

Preferably the compounds show the presence of [(3-hydroxy-4-pyron-2-yl)methyl]-amine units also designated as "MALT").
The MALT units are separated by different aliphatic spacers having or not having a cyclic skeleton.

All the classes of compounds I, II and III, merely by way of example, can be prepared following a known analogous synthetic approach.

The compounds of the present invention and the relative pharmaceutically acceptable salts are preferably used in combination with the DNA demethylating agents and can be used as pharmaceutical compositions together with carriers, stabilizers, diluents or pharmaceutically acceptable excipients.

The compounds and the relative pharmaceutical compositions can be used for the preparation of a medicament.

Use as an antibiotic or for the preparation of a medicament for the treatment of neoplasias is particularly advantageous.

Use for the preparation of a medicament to inhibit the proliferation of neoplastic cells is particularly preferred, in particular for the preparation of a medicament to induce the differentiation of tumour cells in a tumour.

Even more advantageously, they are used for the treatment of primary or secondary cancers, for example rhabdomyosarcoma or cancers of hemopoietic origin, in particular leukemias and lymphomas.

Even more advantageously, they are used for the treatment of sarcomas.

The examples of preparation of the following compounds further illustrate the invention, clearly without limiting the invention to them.

### EXAMPLE 1

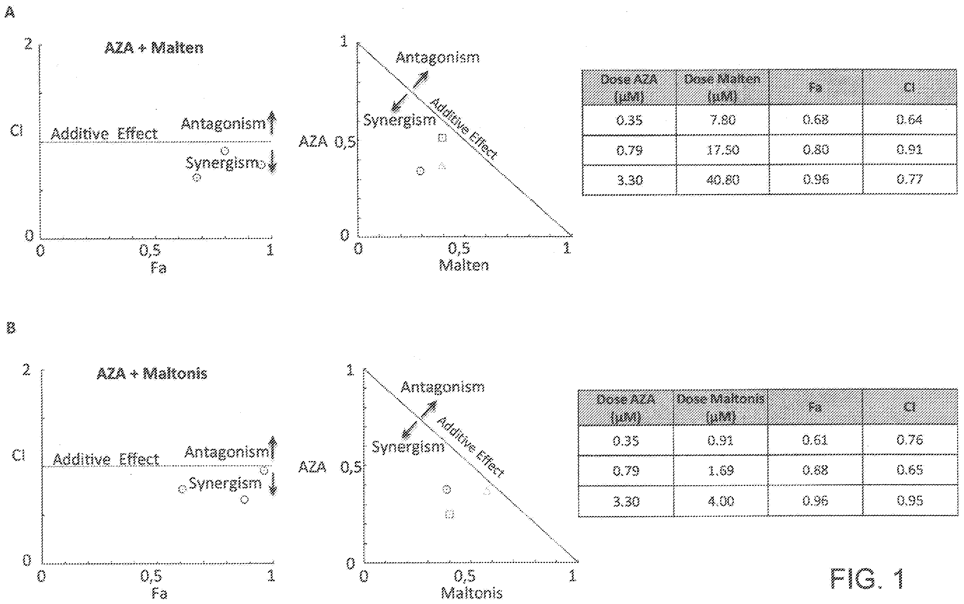

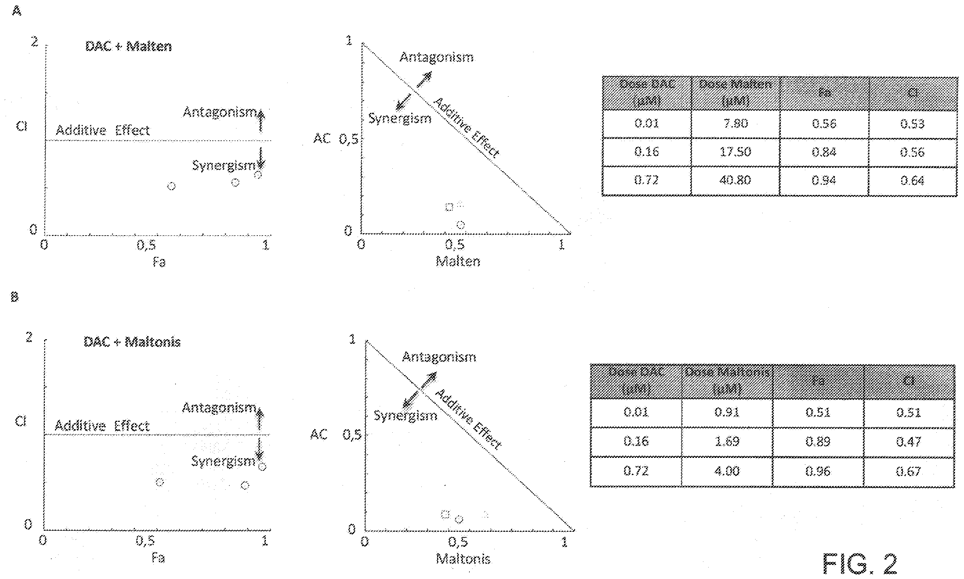

HL-60 cells (promyelocytic leukemia) were treated for 72 hours with different concentrations of 5-aza-cytidine (AZA) and Malten or Maltonis as illustrated in Figure 1 or 5-aza-2'-deoxycytidine (DAC) and Malten or Maltonis as illustrated in Figure 2. At the end of the treatment the cell viability was evaluated by means of Trypan Blue dye exclusion test. The synergic effect of the combined treatments was evaluated by calculating the Fa (Fraction affected) and the Combination Index (CI). The Fa-CI graph and the isobologram, shown in figures 1 and 2, report the values relative to the synergic effect of the combined treatments. The Fa-CI graph derives from the values presented in the corresponding table and highlights the effect, whereas the isobologram expresses the synergy, highlighting the dose. A CI value below 1 indicates synergy. Both the graphs were generated using the CompuSyn software (Chou TC, Pharmacol. Rev. 2006, 58(3):621-681).

In all the cases tested, the synergic biological effect (CI < 1) is clear, in particular using low concentrations of the single molecules used.

### Example 2

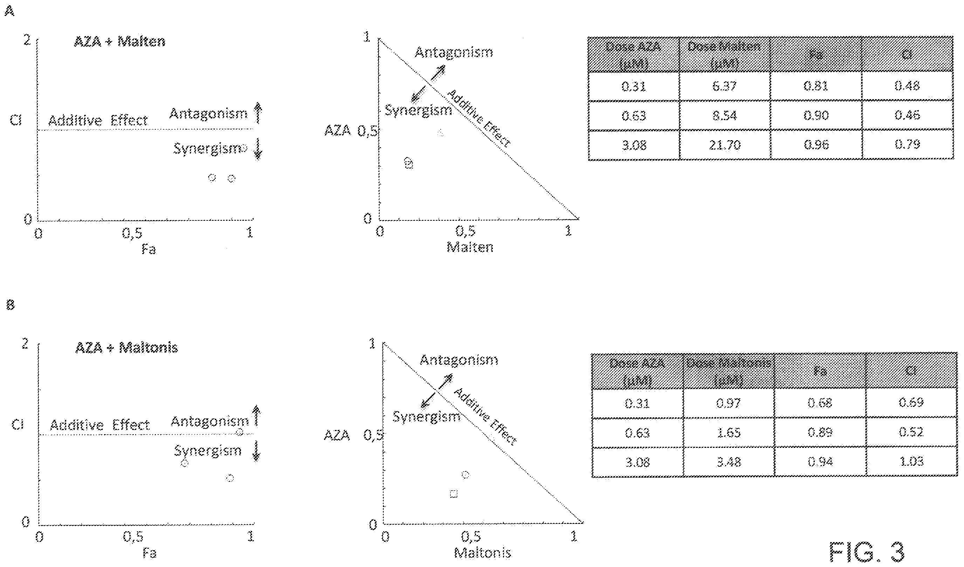

Figure 3 illustrates the synergic effect obtained by means of combined treatments of bis-maltolates (malten or maltonis) with the DNA (AZA) demethylating agent and U937 cells (histiocytic lymphoma). U937 cells were treated for 72 hours with different concentrations of AZA and malten (A) or AZA and maltonis (B) and then evaluated for viability by means of Trypan Blue dye exclusion test. The Fa-CI graphs (left-hand panels) and normalized isobolograms (right-hand panels) show the synergic biological effect. The Fa-CI graphs were derived from the values indicated in the corresponding table. Fa (fraction affected) represents the fraction of cells affected by the treatment. Combination index (CI) <1 indicates synergy. All the experiments were carried out in triplicate and the mean value is displayed. The Fa-CI graphs and the normalized isobolograms were generated using the CompuSyn software (Chou, 2006).

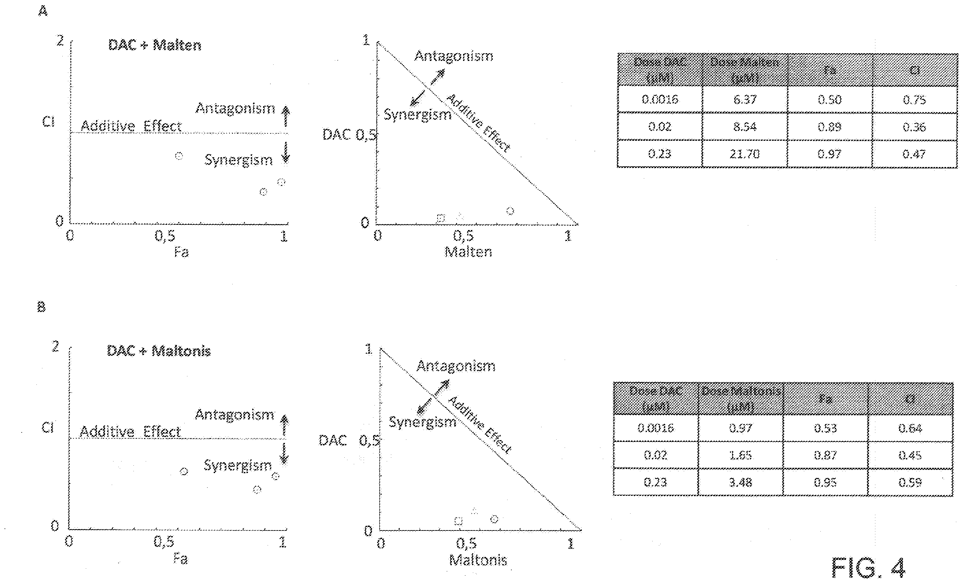

Figure 4 illustrates the synergic effect triggered by combined treatments of bis-maltolates (malten or maltonis) with the DNA (DAC) demethylating agent. U937 cells were treated for 72 hours with different concentrations of DAC and malten (A) or DAC and maltonis (B) and then evaluated for viability by means of Trypan Blue dye exclusion test. The Fa-CI graphs (left-hand panels) and normalized isobolograms (right-hand panels) show the synergic biological effect. The Fa-CI graphs were derived from the values indicated in the corresponding table. Fa (fraction affected) represents the fraction of cells affected by the treatment.

The combination index (CI) <1 indicates an unexpected synergic effect.

All the experiments were performed in triplicate and the mean value is displayed. The Fa-CI graphs and the normalized isobolograms were generated using the CompuSyn software (Chou, 2006).

## Claims

1. - Pharmaceutical composition comprising at least one compound with Formula I: 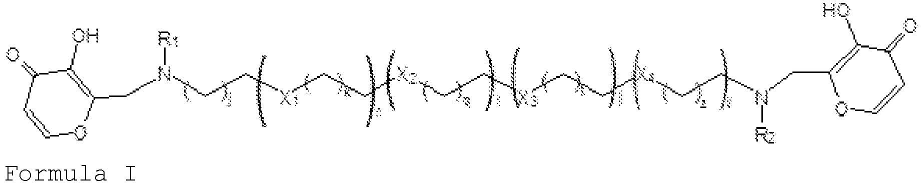 where:
X₁, X₂, X₃, X₄ = NR₃, O
R₃ = H, CₘH₂ₘ₊₁, m = 1, 2, 3, 4, 5, 6
n, f, l, v = 0,1
j, k, q, t, z = 1, 2
R₁, R₂ = CₘH₂ₘ₊₁, m = 1, 2, 3, 4, 5, 6
or alternatively form a cycle and have the following meaning:
R₁, R₂ = 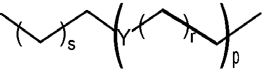 where:
Y = NR₃, O
R₃ = H, CₘH₂ₘ₊₁, m = 1, 2, 3, 4, 5, 6
p= 0, 1, 2, 3, 4;
s= 0, 1, 2
r= 1, 2
and at least one DNA demethylating agent.

2. Pharmaceutical composition comprising at least one compound with formula II: 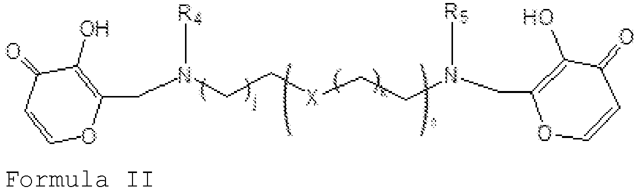 where
X = NR₃, O
R₃ = H, CₘH₂ₘ₊₁, m = 1, 2, 3, 4, 5, 6
n = 0, 1, 2, 3, 4
j, k = 1, 2
R₄, R₅ = CₘH₂ₘ₊₁, m = 1, 2, 3, 4, 5, 6
and one compound comprising at least one DNA demethylating agent for use in the preparation of a medicament.

3. Pharmaceutical composition comprising at least one compound with formula III: 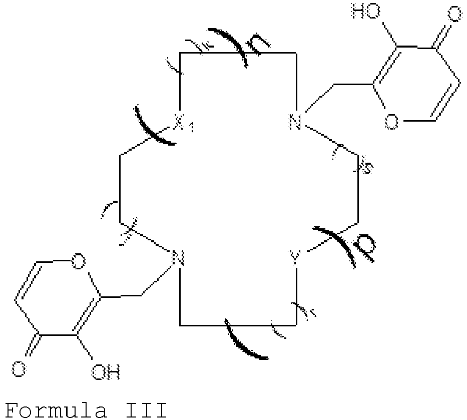 where:
X₁, Y = NR₃, O
R₃ = H, CₘH₂ₘ₊₁, m = 1, 2, 3, 4, 5, 6
n = 0, 1, 2, 3
j, s = 0, 1, 2
P= 0, 1, 2, 3
r, k= 1, 2
and at least one DNA demethylating agent for use in the preparation of a medicament.

4. Pharmaceutical composition comprising [N, N'-bis [(3-hydroxy-4-Pyron-2-yl) methyl] -N, N'-dimethylethylenediamine] or [4(N), 10(N) -bis [(3-hydroxy-4-Pyron-2-yl) methyl]-1,7-dimethyl-1, 4, 7, 10 tetraazacyclododecane] and at least one compound comprising a DNA demethylating agent.

5. Pharmaceutical composition according to any one of the preceding claims, **characterized in that** said at least one demethylating agent is an analogue of cytidine.

6. Pharmaceutical composition according to any one of the preceding claims, **characterized in that** said at least one demethylating agent is 5-aza-cytidine (AZA) or 5-aza-2'-deoxycytidine (DAC).

7. Pharmaceutical composition according to any one of the claims from 1 to 6 in combination with at least one out of a carrier, a stabilizer, a diluent or a pharmaceutically acceptable excipient.

8. Pharmaceutical composition according to any one of the claims from 1 to 7 for use as a medicament.

9. Pharmaceutical composition according to any one of the claims from 1 to 7 for use as a combined preparation for simultaneous, separate or sequential use to inhibit the proliferation of neoplastic cells.

10. Pharmaceutical composition according to any one of the claims from 1 to 7 for use as a combined preparation for simultaneous, separate or sequential use to induce the differentiation of tumour cells in a tumour.

11. Pharmaceutical composition according to any one of the claims from 1 to 7 for use as a combined preparation for simultaneous, separate or sequential use for the treatment of primary or secondary cancers.

12. Pharmaceutical composition according to any one of the claims from 1 to 7 for use as a combined preparation for simultaneous, separate or sequential use for the treatment of rhabdomyosarcoma or tumours of hematopoietic origin, in particular, leukemias and lymphomas.

13. Pharmaceutical composition according to any one of the claims from 1 to 7 for use as a combined preparation for simultaneous, separate or sequential use for the treatment of sarcomas.

## Patentansprüche

1. Pharmazeutische Zusammensetzung, umfassend wenigstens eine Verbindung der Formel I: 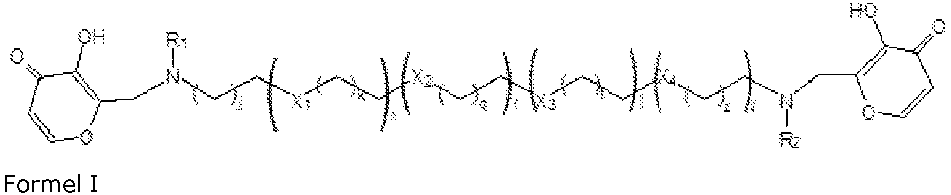 wobei:
X₁, X₂, X₃, X₄ = NR₃, O;
R₃ = H, CₘH₂ₘ₊₁, m = 1, 2, 3, 4, 5, 6;
n, f, l, v = 0, 1;
j, k, q, t, z = 1, 2;
R₁, R₂ = CₘH₂ₘ₊₁, m = 1, 2, 3, 4, 5, 6;
oder alternativ dazu einen Ring bilden und die folgende Bedeutung haben:
R₁, R₂ = 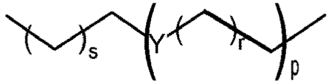 wobei:
Y = NR₃, O;
R₃ = H, CₘH₂ₘ₊₁, m = 1, 2, 3, 4, 5, 6;
p = 0, 1, 2, 3, 4;
s = 0, 1, 2;
r = 1, 2;
und wenigstens ein DNA-Demethylierungsmittel.

2. Pharmazeutische Zusammensetzung, umfassend wenigstens eine Verbindung der Formel II: 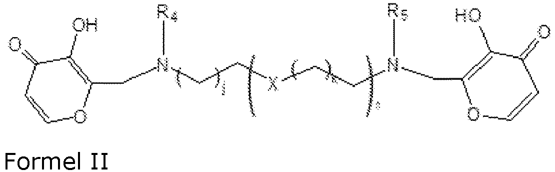 wobei:
X = NR₃, O;
R₃ = H, CₘH₂ₘ₊₁, m = 1, 2, 3, 4, 5, 6;
n = 0, 1, 2, 3, 4;
j, k = 1, 2;
R₄, R₅ = CₘH₂ₘ₊₁, m = 1, 2, 3, 4, 5, 6;
und eine Verbindung, die wenigstens ein DNA-Demethylierungsmittel umfasst, zur Verwendung bei der Herstellung eines Medikaments.

3. Pharmazeutische Zusammensetzung, umfassend wenigstens eine Verbindung der Formel III: 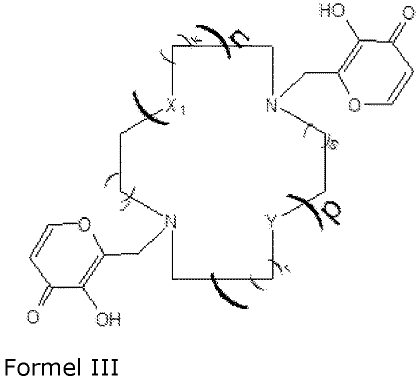 wobei:
X₁, Y = NR₃, O;
R₃ = H, CₘH₂ₘ₊₁, m = 1, 2, 3, 4, 5, 6;
n = 0, 1, 2, 3;
j, s = 0, 1, 2;
p = 0, 1, 2, 3;
r, k = 1, 2;
und wenigstens ein DNA-Demethylierungsmittel zur Verwendung bei der Herstellung eines Medikaments.

4. Pharmazeutische Zusammensetzung, umfassend [N,N'-Bis[(3-hydroxy-4-pyron-2-yl)methyl]-N,N'-dimethylethylendiamin] oder [4(N),10(N)-Bis[(3-hydroxy-4-pyron-2-yl)methyl]-1,7-dimethyl-1,4,7,10-tetraazacyclododecan] und wenigstens eine Verbindung, die ein DNA-Demethylierungsmittel umfasst.

5. Pharmazeutische Zusammensetzung gemäß einem der vorstehenden Ansprüche, **dadurch gekennzeichnet, dass** das wenigstens eine Demethylierungsmittel ein Analogon von Cytidin ist.

6. Pharmazeutische Zusammensetzung gemäß einem der vorstehenden Ansprüche, **dadurch gekennzeichnet, dass** es sich bei dem wenigstens einen Demethylierungsmittel um 5-Azacytidin (AZA) oder 5-Aza-2'-desoxycytidin (DAC) handelt.

7. Pharmazeutische Zusammensetzung gemäß einem der Ansprüche 1 bis 6 in Kombination mit wenigstens einem aus einem Träger, einem Stabilisator, einem Verdünnungsmittel oder einem pharmazeutisch annehmbaren Arzneimittelhilfsstoff.

8. Pharmazeutische Zusammensetzung gemäß einem der Ansprüche 1 bis 7 zur Verwendung als Medikament.

9. Pharmazeutische Zusammensetzung gemäß einem der Ansprüche 1 bis 7 zur Verwendung als Kombinationspräparat für die gleichzeitige, getrennte oder sukzessive Verwendung zur Hemmung der Vermehrung von neoplastischen Zellen.

10. Pharmazeutische Zusammensetzung gemäß einem der Ansprüche 1 bis 7 zur Verwendung als Kombinationspräparat für die gleichzeitige, getrennte oder sukzessive Verwendung zur Induktion der Differenzierung von Tumorzellen in einem Tumor.

11. Pharmazeutische Zusammensetzung gemäß einem der Ansprüche 1 bis 7 zur Verwendung als Kombinationspräparat für die gleichzeitige, getrennte oder sukzessive Verwendung zur Behandlung von primären oder sekundären Krebserkrankungen.

12. Pharmazeutische Zusammensetzung gemäß einem der Ansprüche 1 bis 7 zur Verwendung als Kombinationspräparat für die gleichzeitige, getrennte oder sukzessive Verwendung zur Behandlung von Rhabdomyosarkomen oder Tumoren hämatopoetischer Herkunft, insbesondere Leukämien und Lymphomen.

13. Pharmazeutische Zusammensetzung gemäß einem der Ansprüche 1 bis 7 zur Verwendung als Kombinationspräparat für die gleichzeitige, getrennte oder sukzessive Verwendung zur Behandlung von Sarkomen.

## Revendications

1. Composition pharmaceutique comprenant au moins un composé de formule I : 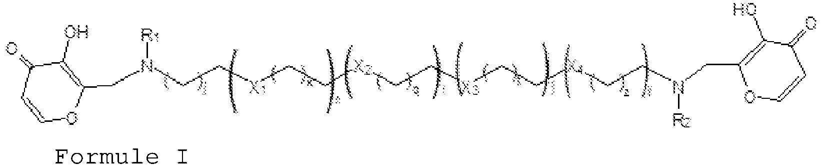 dans laquelle :
X₁, X₂, X₃, X₄ = NR₃, O
R₃ = H, CₘH₂ₘ₊₁, m = 1, 2, 3, 4, 5, 6
n, f, 1, v = 0, 1
j, k, q, t, z = 1, 2
R₁, R₂ = CₘH₂ₘ₊₁, m = 1, 2, 3, 4, 5, 6
ou, en variante, forment un cycle et ont la signification suivante :
R₁, R₂ = 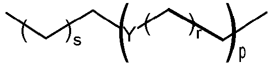 dans laquelle :
Y = NR₃, O
R₃ = H, CₘH₂ₘ₊₁, m = 1, 2, 3, 4, 5, 6
p = 0, 1, 2, 3, 4 ;
s = 0, 1, 2
r = 1, 2
et au moins un agent déméthylant de l'ADN.

2. Composition pharmaceutique comprenant au moins un composé de formule II : 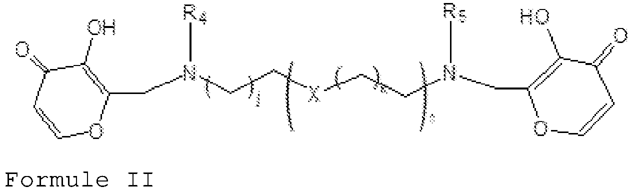 dans laquelle :
X = NR₃, O
R₃ = H, CₘH₂ₘ₊₁, m = 1, 2, 3, 4, 5, 6
n = 0, 1, 2, 3, 4
j, k = 1, 2
R₄, R₅ = CₘH₂ₘ₊₁, m = 1, 2, 3, 4, 5, 6
et un composé comprenant au moins un agent déméthylant de l'ADN destinée à être utilisée dans la préparation d'un médicament.

3. Composition pharmaceutique comprenant au moins un composé de formule III : 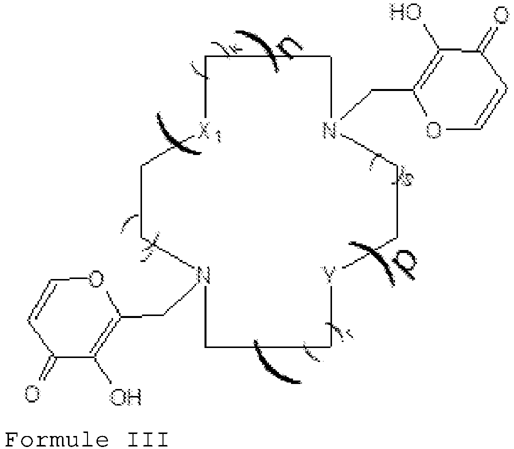 dans laquelle :
X₁, Y = NR₃, O
R₃ = H, CₘH₂ₘ₊₁, m = 1, 2, 3, 4, 5, 6
n = 0, 1, 2, 3
j, s = 0, 1, 2
P = 0, 1, 2, 3
r, k = 1, 2
et au moins un agent déméthylant de l'ADN destinée à être utilisée dans la préparation d'un médicament.

4. Composition pharmaceutique comprenant de la [N,N'-bis[(3-hydroxy-4-pyron-2-yl)méthyl]-N,N'-diméthyléthylènediamine] ou du [4(N),10(N)-bis[(3-hydroxy-4-pyron-2-yl)méthyl]-1,7-diméthyl-1,4,7,10-tétraazacyclododécane] et au moins un composé comprenant un agent déméthylant de l'ADN.

5. Composition pharmaceutique selon l'une quelconque des revendications précédentes, **caractérisée en ce que** ledit au moins un agent déméthylant est un analogue de la cytidine.

6. Composition pharmaceutique selon l'une quelconque des revendications précédentes, **caractérisée en ce que** ledit au moins un agent déméthylant est la 5-aza-cytidine (AZA) ou la 5-aza-2'-désoxycytidine (DAC).

7. Composition pharmaceutique selon l'une quelconque des revendications 1 à 6 en combinaison avec au moins un composant parmi un véhicule, un stabilisant, un diluant ou un excipient pharmaceutiquement acceptable.

8. Composition pharmaceutique selon l'une quelconque des revendications 1 à 7 destinée à être utilisée comme médicament.

9. Composition pharmaceutique selon l'une quelconque des revendications 1 à 7 destinée à être utilisée en tant que préparation combinée pour une utilisation simultanée, séparée ou séquentielle afin d'inhiber la prolifération des cellules néoplasiques.

10. Composition pharmaceutique selon l'une quelconque des revendications 1 à 7 destinée à être utilisée en tant que préparation combinée pour une utilisation simultanée, séparée ou séquentielle afin d'induire la différenciation des cellules tumorales dans une tumeur.

11. Composition pharmaceutique selon l'une quelconque des revendications 1 à 7 destinée à être utilisée en tant que préparation combinée pour une utilisation simultanée, séparée ou séquentielle pour le traitement des cancers primitifs ou secondaires.

12. Composition pharmaceutique selon l'une quelconque des revendications 1 à 7 destinée à être utilisée en tant que préparation combinée pour une utilisation simultanée, séparée ou séquentielle pour le traitement du rhabdomyosarcome ou des tumeurs d'origine hématopoïétique, en particulier des leucémies et des lymphomes.

13. Composition pharmaceutique selon l'une quelconque des revendications 1 à 7 destinée à être utilisée en tant que préparation combinée pour une utilisation simultanée, séparée ou séquentielle pour le traitement des sarcomes.
